Europäisches Patentamt

European Patent Office

Office européen des brevets

(11) Veröffentlichungsnummer: **0 463 194 A1**

# EUROPÄISCHE PATENTANMELDUNG

(21) Anmeldenummer: **90111954.5**

(22) Anmeldetag: **23.06.90**

(51) Int. Cl.⁵: **C08G 63/08**, A61K 47/48

(43) Veröffentlichungstag der Anmeldung:
**02.01.92 Patentblatt 92/01**

(84) Benannte Vertragsstaaten:
**DE**

(71) Anmelder: **BOEHRINGER INGELHEIM KG**
**Postfach 200**
**W-6507 Ingelheim am Rhein(DE)**

(72) Erfinder: **Stricker, Herbert Prof. Dr.**
**Richard-Lenel-Weg 13**
**W-6903 Neckargemünd(DE)**
Erfinder: **Bendix, Dieter Dr.**
**Veith-Stoss-Strasse 17**
**W-6507 Ingelheim am Rhein(DE)**

(54) **Verfahren zur Herstellung von Poly-(D,L-lactid) und seine Verwendung als Wirkstoffträger.**

(57) D,L-Lactid wird in Gegenwart von Katalysatoren und definierter Mengen Polymilchsäure zu Poly(D,L-lactid) polymerisiert. Das vorzugsweise niedermolekulare Produkt soll als implantierbarer Träger pharmakologischer Wirkstoffe eingesetzt werden.

Die Erfindung betrifft ein neues verfahren zur Herstellung niedrigviskoser Poly-(D,L-lactide) sowie deren Verwendung als Wirkstoffträger für Arzneimittel.

Aus dem Stand der Technik sind zahlreiche Wirkstofffreigabesysteme aus resorbierbaren Polymeren bekannt, die injizierbar oder implantierbar sind. Solche Systeme sind immer dann bevorzugt, wenn ein Wirkstoff über einen längeren Zeitraum verabreicht werden soll und eine perorale Applikation nicht möglich bzw. nicht ausreichend verläßlich ist. Ein wirkstoffhaltiges resorbierbares Implantat ist gegenüber der oralen Applikation auch dann bevorzugt, wenn der Wirkstoff im Körper innerhalb eines eng begrenzten Bereichs abgegeben werden soll, z.B. bei der Behandlung von Tumoren mit hochwirksamen Cytostatica.

Implantierbare Wirkstoffträger sollten die folgenden Kriterien erfüllen:

Der Wirkstoff sollte über einen längeren Zeitraum in gleichbleibender Geschwindigkeit abgegeben werden und das Implantat sollte in einem angemessenen Zeitraum abgebaut werden, so daß eine operative Entfernung des Implantats nach der Wirkstofffreigabe entfällt. Es ist hierbei von besonderem Vorteil, daß das Implantat während der Phase, in der der Wirkstoff abgegeben wird, in seiner geometrischen Struktur beständig ist und anschließend relativ schnell abgebaut wird. Hierdurch wird gewährleistet, daß die zu Beginn der Wirkstofffreigabe vorhandene Freisetzungsrate während der Behandlungsdauer nahezu konstant bleibt. Wichtige Parameter für diese Eigenschaften sind die Quellfähigkeit des Implantats in einem wäßrigen Medium (Wasseraufnahme), die Molmassenabnahme des verwendeten Polymeren und die Massenabnahme des Implantats selbst.

Verfahren zur Herstellung von Poly-(D,L-lactid) - so z.B. durch Polymerisation von Lactid in Gegenwart geeigneter Polymerisationskatalysatoren oder durch Polykondensation von Milchsäure - gehören zum Stand der Technik.

Überraschenderweise wurde gefunden, daß ein implantierbarer Wirkstoffträger, der aus einem Poly-(D,L-lactid) geringer Viskosität, das nach dem folgenden Verfahren hergestellt wird, besondere Vorteile aufweist.

Das erfindungsgemäße Verfahren ist dadurch charakterisiert, daß D,L-Lactid in Gegenwart geeigneter Polymerisationskatalysatoren unter Zusatz definierter Anteile von Polymilchsäure polymerisiert wird.

Unter "Polymilchsäure" werden hier Polymere verstanden, die aus Milchsäureeinheiten aufgebaut sind und einen niedrigen Polymerisationsgrad aufweisen. Solche Polymilchsäuren werden üblicherweise durch Kondensation von Milchsäuren hergestellt, werden aber auch bei der ringöffnenden Polymerisation von Lactiden unter geeigneten Bedingungen erhalten.

Geeignete Polymerisationskatalysatoren sind aus dem Stand der Technik bekannt, so z.B. organische und anorganische Zinnverbindungen, wie beispielsweise Zinnlactat, Zinntartrat, Zinnoxalat, Zinndicaprylat, Zinndilaurat, Zinndipalmitat, Zinnchlorid u.a.. Besonders bevorzugt ist Zinndioctoat, besser bezeichnet als Zinn-di-(2-ethyl-hexanoat).

Der Zusatz an Polymilchsäure - bevorzugt Poly(D,L-Milchsäure) - liegt zwischen 5 und 40, vorzugsweise zwischen 20 und 30 %, bezogen auf das eingesetzte D,L-Lactid. Erfindungsgemäß wird unter Polymilchsäure Poly-D-, Poly-L- oder Poly-D,L-milchsäure verstanden. Die eingesetzte Polymilchsäure sollte ein mittleres Molekulargewicht, bestimmt durch Endgruppentitration, von ca. 1800 bis 2500 (Zahlenmittel) besitzen. Das Gewichtsmittel, bestimmt durch Gelpermeationschromatographie gegen engverteilte Poly-(styrol)-Standards, sollte zwischen 3000 and 4000 liegen, die Polydispersität bei ca. 2,5 bis 3,5.

Nach dem erfindungsgemäßen Verfahren hergestelltes Poly-(D,L-lactid), das eine inhärente Viskosität zwischen 0.15 und 0.25, bevorzugt 0.17 und 0.20 (gemessen in Chloroform bei 25°C, c = 100 mg / 100 ml) aufweist, eignet sich zur Herstellung der erfindungsgemäßen implantierbaren Wirkstofftäger. Diese Implantate zeichnen sich durch eine geringfügige Wasseraufnahme (Quellfähigkeit) und eine geringe Massenabnahme während der ersten 35 Tage nach der Implantation aus. Dies bedeutet eine konstante Freisetzung des Wirkstoffes in dieser Zeit. Im Anschluß daran wird das erfindungsgemäße Implantat rasch abgebaut.

Nach dem erfindungsgemäßen Verfahren hergestellte Polymere und daraus gefertigte Implantate weisen folgende Vorteile auf:

1) niedriger Schmelzbereich < 100°C, bevorzugt 60 - 75°C.

2) niedrige Massetemperatur bei der Extrusion.

3) Masseabnahme:

Ca. 35 Tage keine Abnahme der Masse, dann praktisch exponentiell verlaufende Abnahme der Masse. Dadurch ergibt sich ein auch mechanisch stabiles Implantat mit definierter Geometrie und damit vorhersagbarem, konstantem Freigabeverhalten.

4) Quellverhalten

Im Gegensatz zu Poly-(D,L-lactid-co-glykolid) zeigt das erfindungsgemäß hergestellte Poly-(D,L-lactid) praktisch erst nach 35 Tagen eine Wasseraufnahme, während Poly-(D,L-lactid-co-glycolid) bereits nach 9

2

Tagen große-Mengen Wasser aufnimmt.

Auswirkungen:

    a) s. Pkt 3

    b) verlängerte Stabilität von wasserempfindlichen Wirkstoffen

5) Molekulargewichtsabnahme

Die erfindungsgemäß hergestellten Polymere zeigen erst nach ca. 10 Tagen einen signifikanten Molekulargewichtsabbau und sind demnach besser zur Herstellung von Devices mit konstantem Verhalten geeignet, da der Molekulargewichtsabbau direkt auch das Freigabeverhalten beeinflußt.

6) Änderungen des TG

Die erfindungsgemäß hergestellten Polymere haben ca. 30 Tage einen TG (Glaspunkt) über der Körpertemperatur. Implantate aus diesen Polymeren bleiben also lange formstabil.

Die erfindungsgemäßen Implantate können nach verschiedenen Verfahren hergestellt werden, so z.B. durch Extrusion (A) oder nach der sogenannten "Lösungsmethode" (B).

(A) Poly-(D,L-lactide) mit Viskositäten zwischen 0.15 und 0.25 weisen einen Schmelzbereich unterhalb 100 $^{\circ}$C auf und können somit durch Extrusion verarbeitet werden. Hierzu wird das Polymer in Form eines Pulvers oder Granulats mit dem Wirkstoff vermischt. Falls es gewünscht wird, können weitere Hilfsstoffe, z.B. wasserlösliche Porenbildner in Form von Lactose, zugesetzt werden. Anschließend wird die Mischung zu Stangen extrudiert, die dann zu Stäbchen geschnitten werden. Beispielsweise beträgt die Länge eines Stäbchens ca. 3 cm und der Durchmesser ca. 2,8 mm.

(B) Bei der Lösungsmethode wird das Polymer zusammen mit dem Wirkstoff und eventueller Hilfs- und Zusatzstoffe gelöst, zu Filmen gegossen und weiterverarbeitet. Hierbei sind folgende Ausführungsformen denkbar:

A) massive Stäbchen,

B) gerollte Filme,

C) ummantelte Stäbchen,

D) schlauchförmige Körper,

E) ummantelte schlauchförmige Körper

Alle Ausführungsformen des erfindungsgemäßen Implantats können mehrschichtig aufgebaut und beispielsweise nach folgendem Verfahren hergestellt werden:

In dem gelösten Polymer, z.B. mit Essigester, Aceton oder einem anderen pharmakologisch unbedenklichen Lösungsmittel als Lösungsmittel, wird der Wirkstoff suspendiert. Wenn es gewünscht wird, können dem gelösten Polymer neben dem Wirkstoff noch weitere pharmazeutische Hilfsstoffe, wie z.B. wasserlösliche Porenbildner, zugesetzt werden. Danach wird die Suspension auf einer Fläche ausgegossen und zu einem Film getrocknet. Hierbei werden die Trocknungsbedingungen so eingestellt, daß die gewünschte Restmenge an Lösungsmittel in dem Polymer verbleibt, im allgemeinen zwischen 1 und 8 %, bevorzugt zwischen 4 und 7 %. Die getrockneten Filme haben eine Schichtdicke zwischen 30 und 1000 $\mu$, bevorzugt ca. 100 $\mu$. Geräte und Verfahren zur Herstellung solcher Filme sind dem Fachmann bekannt und bedürfen keiner weiteren Ausführungen. Es ist selbstverständlich, daß der Trocknungsprozeß mit einer gewissen Sorgfalt (langsam, geringe Temperatur- und Vakuum-Feuchtigkeitsschwankungen) durchgeführt werden muß, damit die Filme plan bleiben.

Mehrschichtige Filme können durch ein erneutes Aufbringen von Polymerlösung (mit oder ohne Wirkstoff) erhalten werden.

Nachdem der Film getrocknet ist, wird er zu Stäbchen der gewünschten Länge geschnitten.

Stäbchen des Typs B bestehen aus einem oder mehreren aufgerollten ein- oder mehrschichtigen Polymerfilmen. Das erfindungsgemäße Implanat des Typs B wird ebenfalls aus wirkstoffhaltigen Filmen hergestellt, wobei jedoch die Dicke der Filme wesentlich geringer ist, im allgemeinen zwischen 30 und 500 $\mu$m, bevorzugt 70 bis 90 $\mu$m. Nach dem Trocknen werden die Filme geschnitten und zu Stäbchen gewünschten Durchmessers, bis ca. 3 mm, gerollt, die dann auf die gewünschte Länge geschnitten werden. Die Stäbchen können so aufgerollt werden, daß der Kern einen Hohlraum enthält. Bei dem Laminat des Typs B können auch mehrere Filme übereinandergelegt oder bevorzugt übereinander gegossen werden, die dann zu einem Stäbchen gerollt werden. Durch die Kombination mehrerer Filmschichten können in einfacher Weise Wirkstoffe kombiniert werden und Schichten unterschiedlicher Wirkstoffkonzentration hergestellt werden. Die einzelnen Schichten können verschiedene Freisetzungsgeschwindigkeiten aufweisen.

Neben einer alternierenden Schichtfolge ist es auch möglich, zuerst einen aufgerollten Kern zu bilden und anschließend außen weitere Filmschichten aufzubringen.

Durch die Verwendung von Filmschichten mit unterschiedlicher Freisetzungscharakteristik können mit einem Implantat auch verschiedene Wirkstoffe in zeitlich voher bestimmter Reihenfolge abgegeben werden. Es ist nicht unbedingt erforderlich, daß alle Filmschichten Wirkstoffe enthalten.

Bei der Herstellung des erfindungsgemäßen Implantats des Typs B sollten die Filme einen relativ hohen Gehalt an Restlösungsmittel aufweisen (ca. 10.%), wenn sie gerollt werden. Hierdurch wird vermieden, daß die Filme brüchig werden. Das fertig gerollte Stäbchen wird dann noch einmal einem Trocknungsprozeß unterworfen, um den gewünschten Gehalt an Restlösungsmittel einzustellen.

Die Wirkstofffreisetzung bei den ummantelten Formen C und E erfolgt aufgrund ihrer Konstruktion auf verschiedenen Wegen. Der im Kern der ummantelten Stäbchen der Form C suspendierte Wirkstoff diffundiert durch Poren im Mantel, die durch Herauslösen - z.B. von Laktose - entstehen. Maßgebende Freigabefaktoren sind daher der Beladungsgrad des Mantels und die Partikelgröße der Lactose.

Im Gegensatz zu den ummantelten Formen des Typs C, die einen massiven wirkstoffhaltigen Kern enthalten und von einem "porösen" Mantel umhüllt sind, bestehen die Implantate der Form E aus einem wirkstoffhaltigen Hohlzylinder (Schlauch), dessen Außenfläche von einem wirkstoffundurchlässigen Mantel umhüllt ist.

Bei der Form E kann der im schlauchförmigen Körper suspendierte Wirkstoff - wenn der Mantel porenfrei und undurchlässig ist - nur in den Hohlraum des Körpers (Schlauchs) freigesetzt werden. Bei diesem System werden die mit der Zeit länger werdenden Kanäle, d.h. Diffusionsstrecken, durch die Wirkstoffmenge in einem Segment kompensiert, die umso größer ist, je größer die Entfernung zur Zylinderachse ist.

### Figur A

Figur A zeigt einen Querschnitt durch die erfindungsgemäße Ausführungsform E
Maßgebende Freigabefaktoren sind in diesem Falle - neben dem Polymerabbau und dem Beladungsgrad - die Abmessungen, wie z.B. die Länge und der innere Durchmesser des schlauchförmigen Implantates. Es ist selbstverständlich, daß bei den Implantaten der Form E der Mantel ebenfalls aus einem biologisch abbaubaren Polymer, bevorzugt einem Poly-(D,L-lactid), besteht. Ein wesentlicher Vorteil der so ausgebildeten Implantate ist, daß die Wirkstofffreisetzung nahezu linear erfolgt.

Implantate dieses Typs können auch auf Basis der zuvor beschriebenen Filme hergestellt werden, wobei der äußere Film aus einer wirkstofffreien, wirkstoffundurchlässigen Schicht besteht.

Die nachfolgenden Beispiele erläutern die Erfindung.

Beispiel 1:

In ausgeheizten Zweihalskolben wurden im Handschuhkasten unter trockenem Stickstoff jeweils 40, 35 und 30 g D,L-Lactid, sowie 10 (20 %), 15 (30 %) und 20 g (40 %) Poly-(D,L-milchsäure) eingewogen. Die zugesetzte Polymilchsäure (PMS) hatte folgende analytischen Daten:

$M_n$ = 2000      (Endgruppentitration)

$M_n$ = 1100      (GPC, ND-PS Standards)

$M_w$ = 3400      (GPC, ND-PS Standards)

Gehalt an D,L-Lactide: 2 %      (NMR, $CDCl_3$, 250 MHz)

Wassergehalt:      0,2% (KF-Titration)

Unter trockenem Stickstoff wurde das Gemisch bei 130 °C aufgeschmolzen und dann 25.6 mg Zinnoctoat in toluolischer Lösung als Katalysator zugegeben. Jede Stunde wurde eine Probe gezogen und die inhärente Viskosität (IV) bestimmt
(c = 0.1g/100 ml, Chloroform, 25° C).

| Reaktions-zeit [h] | 20 %PMS IV [dl/g] | 30 % PMS IV [dl/g] | 40 % PMS IV [dl/g] |
|---|---|---|---|
| 1 | .08 | .07 | .08 |
| 2 | .12 | .09 | .07 |
| 3 | .16 | .11 | .09 |
| 4 | .18 | .13 | .09 |
| 5 | .20 | .14 | .10 |
| 6 | .21 | .15 | .11 |
| 7 | .22 | .15 | .11 |

Beispiel 2:

Im Handschuhkasten wurden unter trockenem Stickstoff 400 g D,L-Lactid und 100 g Poly(D,L-milchsäure) in einem Kolben eingewogen.
Das Gemisch wurde unter Rühren und Überdeckung mit trockenem Stickstoff bei 140° C aufgeschmolzen, die Ölbadtemperatur auf 130° C gesenkt und 256 mg Zinnoctoat in toluolischer Lösung als Katalysator zugegeben. Die nach 5 Stunden Polymerisation erhaltene glasartige Polymerschmelze wurde analysiert:

| | |
|---|---|
| Inhärente Viskosität: | O.17 dl/G |
| Restmonomergehalt: | 10.5 % (NMR, $CDCl_2$, 250 MHz) |
| $M_w$ | 13 500 (GPC, PS-Standards, $CHCl_3$) |
| $M_n$ | 6 200 (GPC, PS-Standards, $CHCl_3$) |
| Polydispersität Mw/Mn | 2,2 |

Das Polymer wurde anschließend in der vierfachen Menge Aceton unter kräftigem Rühren in der Siedehitze gelöst, aus Wasser umgefällt und im Vakuumtrockenschrank bei 30° C getrocknet.
Inhärente Viskosität: 0.18 dl/g

Beispiel 3:

In einem helicalen Mixer wurden 7.5 kg D,L-Lactid und 2.5 kg Poly(D,L-milchsäure) vorgelegt und unter Stickstoff aufgeschmolzen. 5 g Zinnoctoat in 20 ml Toluol wurden zugegeben und 5 Stunden bei 170° C Ölbadtemperatur polymerisiert.

Ausbeute: 98 % d. Einsatzes

Das Polymer wurde in 50 l Aceton in der Siedehitze gelöst, filtriert, aus Wasser umgefällt und im Vakuumtrockenschrank bei 38 °C getrocknet.

Ausbeute: 77 % d.E.

Inhärente Viskosität:  0.19 dl/g

Restmonomergehalt:  << 1 % (NMR, $CDCl_3$, 250 MHz)

**Patentansprüche**

1. Verfahren zur Herstellung von Poly-(D,L-lactid) durch ringöffnende Polymerisation von D,L-Lactid in Gegenwart von Polymerisationskatalysatoren, gekennzeichnet durch einen Zusatz definierter Anteile von Polymilchsäure.

2. Verfahren nach Anspruch 1, gekennzeichnet durch einen Anteil von 20 bis 30 Gew.-%. Polymilchsäure bezogen auf eingesetztes D,L-Lactid.

3. Verfahren nach Anspruch 1 oder 2, gekennzeichnet durch einen Anteil von 20 bis 30 Gew.-% Poly-(D,L-milchsäure).

4. Poly-(D,L-lactid) hergestellt durch ringöffnende Polymerisation von D,L-Lactid in Gegenwart von Polymerisationskatalysatoren unter Zusatz von Polymilchsäure.

5. Poly-(D,L-lactid) nach Anspruch 4 gekennzeichnet durch einen Zusatz an Poly-(D,L-milchsäure).

6. Poly-(D,L-lactid) nach Anspruch mit einer inhärenten Viskosität zwischen 0.15 und 0.25 dl/g (gemessen in Chloroform bei 25 °C).

7. Implantierbarer Wirkstoffträger auf der Basis von Poly-(D,L-lactid) gemäß Anspruch 4, 5 oder 6, enthaltend mindestens einen pharmakologisch aktiven Wirkstoff sowie gegebenenfalls übliche Hilfs- und Trägerstoffe.

**Europäisches Patentamt**

**EUROPÄISCHER RECHERCHENBERICHT**

Nummer der Anmeldung

**EP 90 11 1954**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | WO-A-9 001 521  (BATTELLE MEMORIAL INSTITUTE) * Seite 10, Zeile 31 - Seite 12, Zeile 5; Seite 46, Zeile 22 - Seite 47, Zeile 6; Seite 58, Zeile 30 - Seite 59, Zeile 19 * − − − | 1-6 | C 08 G 63/08 A 61 K 47/48 |
| A | DE-A-2 827 289  (ETHICON, INC.) * Anspruch 1; Beispiele 4,5 * − − − | 1 | |
| A | US-A-3 887 699  (S. YOLLES) * Spalte 1, Zeilen 11-16; Spalte 3, Zeile 66 - Spalte 4, Zeile 25; Spalte 5, Zeilen 27-42; Spalte 5, Zeile 61 - Spalte 6, Zeile 9; Spalte 8, Zeile 46 - Spalte 9, Zeile 39; Spalte 10, Zeilen 32-45 * − − − − − | 7 | |

| RECHERCHIERTE SACHGEBIETE (Int. Cl.5) |
|---|
| C 08 G |

Der vorliegende Recherchenbericht wurde für alle Patentansprüche erstellt

| Recherchenort | Abschlußdatum der Recherche | Prüfer |
|---|---|---|
| Den Haag | 31 Januar 91 | KRISCHE D.H.T. |